# EUROPEAN PATENT APPLICATION

(11) **EP 0 654 273 A1**
(43) Date of publication of application: **24.05.1995**
(21) Application number: 94308551.4
(22) Date of filing: 18.11.1994
(51) Int. Cl.: A61K 39/00, A61K 39/02, A61K 39/07, A61K 39/09, A61K 38/50, A61K 9/50, A61K 9/52, A61K 35/74

(54) **Pharmaceutical product and method for treatment**

(30) Priority: 18.11.1993 US 153856; 18.11.1993 US 153857; 24.01.1994 US 185749
(71) Applicant: Leveen, Harry H., Charleston, SC 29407 (US); Leveen, Eric G., Charleston, South Carolina 29047 (US); Leveen, Robert F., Omaha, Nebraska 68114 (US)
(72) Inventor: Leveen, Harry H., Charleston, SC 29407 (US); Leveen, Eric G., Charleston, South Carolina 29047 (US); Leveen, Robert F., Omaha, Nebraska 68114 (US)
(74) Representative: W.P. Thompson & Co.

(57) **Abstract**

Use of a non-enzymatic urease antigen for the manufacture of a medicament for treating and/or preventing, in animals, including humans, infections, by Helicobacter pylori and comparable Helicobacter infections and its use.

## Description

The present invention relates to a urease antigen and to use of a urease antigen for the manufacture of a medicament for treating and/or preventing, in animals, including humans, infections eg. by Helicobacter pylori and comparable Helicobacter infections. The invention also relates to such treatment and/or prevention of such infections.

Knowledge of the role of Helicobacter pylor in causing infection eg. gastritis and peptic ulcer has developed in the last 5 years. The Australian discovery of Helicobacter was at first looked upon with extreme skepticism. (Marshall BOJ, Warren JR., Unidentified Curved Bacilli in the Stomach of Patients with Gastritis and Peptic Ulceration Lancet 1:1311-15, 1984). The confirmation of H. pylori in gastritis and gastric ulcers by others expelled doubts about the association of this organism with gastritis. Also, it was found that Koch's postulates could be fulfilled for H. pylori infections. Gastritis was induced in two human self experiments by the ingestion of live cultures of H. pylori. (Marshall BOJ, Armstrong AJ, dB et al Attempt to Fulfill Koch's Postulates for pyloric Campylobacter MED J AST 142:436-39; 1985; Morris A, Nichols G Ingestion of Campylobacter pyloridis Caused Gastritis and Raised Resting Gastric PH Am J Gastroent 82:192-99, 1987). In one of these cases, the gastritis progessed to chronicity and was present for years. The role which Helicobacter pylori plays in upper gastrointestinal lesions now has firm laboratory and clinical confirmation. Fortuitously, infection with Helicobacter pylori is easily confirmed since the corkscrew shaped organism is a prodigious producer of intracellular and extracellular urease. (Bride EC, Hadfield TL, Heat JR., et al, Campylobacter pyloridis Survival in a Hostile Environment, Am J Gastroent 81:855, 1986). Diagnosis can be established by identifying urease in the gastric juice or by ingestion of C¹⁴ labeled urea, H₂NC¹⁴ONH₂ and the immediate detection of C¹⁴O₂ in the expired air. Gastric urease immediately hydrolyzes the urea to NH₃ and H₂O, and the rapid absorption of ammonia accounts for the appearance of the labeled CO₂ in the expired air. (Novice HB, Gabby G, Leishman G, et al, Two Point Analysis 15 Minute C¹⁴-Urea Breath Test for Diagnosing Helicobacter pylori Infection, Digestion 50:16:21, 1991).

Helicobacter pylori (H. pylori) has been identified in 90% of patients with duodenal ulcers which are not caused by medication with non-steroidal anti-inflammatory agents. (NJ, Rows AJ, Campylobacter pylori and its Role in Peptic Ulcer Disease, Gastroent Cline North Am 19:183-96, 1990). Most people believe that H. pylori is not the sole cause for the ulceration since duodenal ulcer formation does not occur in the absence of acid. (Graham DE Campylobacter pylori and Peptic Ulcer Disease Gastroenterol 96:615-25, 1989). For this reason, H₂ receptor antagonists and omeprazole a proton pump inhibitor are still effective in the treatment of ulcers. Nonetheless, H. pylori must be eliminated if recurrence of duodenal ulcers is to be prevented. An interesting finding suggests the proliferation of H. pylori is dependent on acid produced by the mucosa of the corpus. (Cartooned T, Nimble S, Lethal J Helicobacter pylori in Dyspeptic Patients: Quantitative Association with Severity of Gastritis, Intragastric PH. and Serum Gastrin Concentration, Scand J Gastroent 26(Suppl) 186:124-34, 1991).

The manner in which this inflammatory gastritis and duodenitis is produced and the contribution of ammonia, produced by urease, to the ulcerations and chronic inflammation can be explained as follows. H. pylori generates an envelope of ammonia which hydrochloric acid. (Hazel SI, Lee A, Campylobacter pyloridis, Urease. Hydrogen Ion Back Diffusion and Gastric Ulcers, Lancet 2:15-17, 1986). A mucous coating is intimately adherent to and protects the surface of the mucosal cell. Mucin is soluble in alkaline solutions and precipitates in acid solution. The precipitation of mucous on the cell surface of the gastric mucosa prevents the mucosa from autodigestion and answers the age old question of why the stomach does not digest itself. Loss of the mucous barrier exposes the mucosa to acid-pepsin digestion. For this reason, peptic ulcers occur at the transition point between alkaline intestinal content and acid gastric content. This junction is usually in the first portion of the duodenum in the common duodenal ulcer. In the presence of excessive acid secretion, as in Zollinger Ellison Syndrome, this junction is displaced further down the intestinal tract and ulceration in the second or third part of the duodenum is not uncommon. Ammonia produced from urea splitting Helicobacter solubilizes the gastric mucin and allows for digestion of the mucosa. The toxic effects of ammonia may account for the inflammatory process seen deep the gastric mucosa. It is the mucolytic effects of H. pylori which account for the ulcerogenic effect. (J, Peru A, Guarantee RLL, Marshall BOJ, et al, Mucolytic effects of Helicobacter pylori, Scand J Gastroent 26(Suppl) 187:47-55, 1991). Thus, the destructive properties of H. pylori may be solely due to ammonia as has been found in infections with other urea splitting bacteria. (LeVeen HHS, Falk G, Boric B, et al, Chemical Acidification of Wounds An Adjuvant to Healing and the Unfavorable Action of Alkalinity and Ammonia, NA Surg 178:745-574, 1973). Some years ago, McLaren, in England, found that necrotizing suppurative pyelonephritis could be produced in guinea pigs by the intravenous injection of Proteus mirabilis. He was able to prove this destructive lesion was caused by urea splitting since a chance laboratory mutation yielded a strain of proteus immunology identical to the original but producing no urease. Injection of this organism did not produce pyelonephritis. (McLaren DM, Significance of Urease in Protease-Pyelonephritis: A Bacteriologic Study, Path 96:45-56 1968). A similar experiment has been performed with a non-urease producing mutant of H. pylori. This mutant when administered to gnotobiotic piglets did not induce the gastritis which resulted from the ingestion of the original urea splitting strain. (S, Eaton AK, Rings DM et al, Gastritis Induced by Helicobacter pylori in Gnotobiotic Piglets, RID (Suppl 8) 13:S681-S685, 1991). The virulence of H. pylori is ascribed to urease. (Filius M, Ode G, G, et al, In vitro Inhibition of Helicobacter pylori Urease:Biochemical and Ultrastructure Analysis. Europe J Clin Invest 21:551-557, 1991). Since several other animal models have been described (Fox JAG., Core P., et al, Helicobacter-Associated pylori Gastric Infection in Gnotobiotic Beagle Dogs, Infect. & Immun 99:352-361, 1990; Radin M.J., Eaton K.A., et al., Generation of Ammonia and Mucosal Lesion Formation Hydrolysis of Urease in the Rat Stomach, J. Clin. Gast. 12(Suppl. 1):S104-S109, 1990; Fox J.G., Lee A., et al, Helicobacter felis Gastritis in Gnotobiotic Rats: an Animal Model of Helicobacter pylori Gastritis Infection & Immunology 59:785-791, 1991), the role played by urease in the pathogenesis of the gastritis can be confirmed in the laboratory.

It has been found that control of H. pylori with triple therapy (bismuth, tetracycline and metronidazole) significantly reduces the frequent incidence of ulcer recurrence upon cessation of therapy with H₂ blockers and omeprazole. (Rauws EAJ, Tytgat GNJ, Cure of Duodenal Ulcer Associated with Eradication of Helicobacter pylori, Lancet 335:11233-11235, 1990). However, triple therapy is not without its own complications and the requirement of strict adherence to a difficult medication schedule makes compliance a serious problem. In addition, resistance of H. pylori to metronidazole is not infrequent. Such resistance strains of H. pylori are being increasingly encountered in 70%. (Goodwin, Marshall, Blincow, et al, Prevention of Nitroimidazole Resistance in *Campylobacter pylori* by Co-administration of Bismuth Subcitrate: Clinical and in vitro Studies, J Clin Pathol 41:207-210, 1988). Therefore, non-antibiotic therapy for this problem of H. pylori infections would be ideal, especially since treatment is prolonged. Although gastritis is frequently associated with peptic ulceration (Sipponen P, Helicobacter pylori9464-9469 and Chronic Gastritis: an Increased Risk of Peptic Ulcer? A Review, Scand J Gastroenterol 26(suppl) 187:6-10, 1991), the recurrence rate in simple duodenal ulcers seems to indicate that eradication of H. pylori is essential if relapse is to be prevented, (Graham DY, Lew GM, Klein PD, et al Effect of Treatment of Helicobacter pylori Infection on the Long-term Recurrence of Gastric or Duodenal Ulcers: A Randomized, Controlled Study, Ann Int Med 116:705-708, 1992). Chronic H. pylori infection have been shown to predispose to gastric cancer. (Recavarren-Arce S, Leon-Barua R Cok J, et al, Helicobacter pylori and Progressive Gastric Pathology That Predisposes to Gastric Cancer, Scand J Gastroenterol 26(suppl) 181:51-57, 1991). It has been found to be associated with abdominal discomfort in both adults and children, even in the absence of peptic ulcer. (Maaroos HI, Rago T, Sipponen P, et al, Helicobacter pylori and Gastritis in Children with Abdominal Complaints, Scand J. Gastrienterol 26 suppl 186:95-99, 1991).

Briefly stated, in one aspect of the invention, a method is provided for immunizing animals and man against the effects of acid urease, produced by Helicobacter pylori e.g. colonizing the gastrointestinal tract.

According to one aspect of the invention there is provided a method for treating and/or preventing, in animals including humans, infections by Helicobacter pylori or comparable Helicobacter infections by immunization with a non-enzymatic urease antigen.

The present invention also comprises use of a non-enzymatic urease antigen for the manufacture of a medicament for treating and/or preventing, in animals, including humans, infections, by Helicobacter pylori and comparable Helicobacter infections.

The non-enzymatic urease antigen may be an acid urease of bacterial origin or plant origin.

Preferably the urease is inactivated by covalent bonding to increase molecular weight thereof.

Preferably the antigen is a glutaraldehyde or formaldehyde inactivated acid urease and may be prepared by reacting acid urease with glutaraldehyde to form covalent bonds therebetween.

In one preferred embodiment jack bean urease is inactivated by covalent bonding to produce said non-enzymatic urease antigen wherein said non-enzymatic urease antigen has a mean molecular weight ranging up to 2,000,000 Daltons, preferably in the range of 250,000 to 2,000,000 Daltons.

In another preferred embodiment, urease produced by a microorganism of the genus Lactobacillus is inactivated by covalent bonding to produce said non-enzymatic urease antigen wherein said non-enzymatic urease antigen has a mean molecular weight ranging up to 2,000,000 Daltons, preferably in the range of 250,000 to 2,000,000 Daltons.

The micro organism is preferably one of Lactobacillus fermentum, Lactobacillus reuteri and Lactobacillus ruminis, more preferably Lactobacillus fermentum TK 1214. Another preferred microorganism is Bacillus TB-90.

In another preferred embodiment urease produced by a microorganism of the genus Streptcooccus is inactivated by covalent bonding to produce said non-enzymatic urease antigen wherein said non-enzymatic urease antigen has a molecular weight ranging up to 2,000,000 Daltons preferably in the range of 250,000 to 2,000,000 Daltons.

The microorganism is preferably one of Streptococcus bovis, Streptococcus Reuteri and Stretpococcus salivarius.

Since most ureases encountered in plants and bacteria perform optimally in a neutral or alkaline solution, it is difficult to hypothesize why H. pylori is able to colonize and invade the stomach which is inhospitable because of its acid environment. Hydrochloric acid is secreted by the gastric cells in the fundus at a pH of 1, which is closely equivalent to a 0.1 normal solution of hydrochloric acid. It could be expected that the urease would be relatively inactive at the acid concentrations present in most stomachs. This colonization is partly explained by the fact that urease produced by H. pylori performs optimally in an acid pH. Also, unlike most ureases the Km of the reaction is 0.3 + - 0.1 nM (Dunn BE, Campbell GP, Perez-Perez GI, Blaser MJ Purification and Characterization of Urease from Helicobacter pylori J Biology Chemistry 1990; 265:946469), which brings about the complete conversion of urea to ammonia in very dilute concentrations such as those which are encountered in blood and in gastric content. Urea, in fermented liquors such as beer, are undesirable because they complex with alcohol and undesirably alter the taste and color of the final product. Japanese brewers have utilized urease to remove the urea from the fermentation mixture. However, they require a urease which functions optimally in an acid environment and which is not be appreciably inactivated by the alcohol formed in the fermentation process. They therefore sought bacteria which would produce such a urease. Such a product is mentioned in U.S. patent No. 4,753,882, issued on June 28, 1988, which is assigned to Sapporo Breweries. Its use to remove urea during the fermentation of beer is described in detail. During this fermentation amino acids are liberated. One amino acid, arginine, is rapidly converted to urea by arginase. The urease converts this urea to ammonia which is more easily removed. In the fermentation of whiskey a similar situation arises. U.S. patent No. 5,006,468, issued April 9, 1991 and assigned to Santori Ltd., describes the use of a similar acid urease to remove the ammonia. These acid ureases have a low Kₘ similar to that produced in H. pylori which favors the total conversion of urea to ammonia at low urea concentrations. Similarly U.S. Patent No. 4,970,153, issued on Nov. 13, 1990 and assigned to Nagase Biochemicals Ltd., describes a method for producing an acid urease from Lactobacillus fermentum. This urease has its optimum activity at pH 3. It suggests the use of this enzyme for the removal of urea from an aqueous solution. U.S. Patent No. 5,093,255 describes an acid urease produced by Lactobacillus or Streptococcus. All of the above mentioned acid ureases are used to remove unwanted dissolved urea from liquids of varying types. These acid ureases have a Kₘ of less than 1 which is similar that of H. Pylori.

The gene composition and the sequencing of amino acids for most urease bacteria has been described. (Walz S.E., Wray S.K., Hull S.I., Hull R.A., Multiple Proteins Encoded within the Urease Gene Complex of Proteus mirabilis, J. of Bacteriol. 170(3):1027-1033 1988; Sriwanthana B., Island M.D., Mobley H.L.T., Sequence of the Proteus mirabilis urease accessory gene *ureG*, Gene 129:103-106, 1993; Sakaguchi K., Mitsui K., Nakai N., Kobashi K., Amino Acid Sequence around a Cysteine Residue in The Active Center of Jack Bean Urease, J Biochem (96):73-79, 1984; Hu L., Foxall P.A., Russel R., Mobley H.L.T., Purification of Recombinant *Helicobacter pylori* Urease Apoenzyme Encoded by *ureA and ureB*, Infection and Immunology 60(7):2657-2666, 1992; Lee M.H., Mulrooney S.B., Renner M.J., Markowicz Y. Hausinger R.P., *Klebsiella aerogenes* Urease Gene Cluster: Sequence of ureD and Demonstration that Four Accessory Genes (*ureD, ureE, ureF, and ureG*) Are Involved in Nickel Metallocenter Biosynthesis, J. of Bacteriol 174(13):4324-4330, 1992). There is a great similarity in jack bean urease found in plants and the H. pylori urease in spite of their wide genetic divergence. It is possible to insert the genes for H. pylori urease into E. Coli using recombinant DNA techniques. If only the genes for ureA and ureB are inserted, a type of urease is produced which has the major structure of urease but has no nickel binding site and is non-enzymatic. Enzymatic urease requires accessory genes besides ureA and ureB. Antibodies produced by injection of this nonenzymatic urease molecule fail to inactivate urease produced by H. pylori, and immunization with this antigen will not completely protect laboratory animals against colonization by H. pylori. Because urease is toxic and produces ammonia intoxication in laboratory animals and man, a non-enzymatic urease antigen is required for immunization. The production of such a non-enzymatic urease antigen is described in U.S. patent No. 4,837,017, Urease Antigen Product and Process, issued June 6, 1989, and which is incorporated herein by reference. An inactive urease antigen of acid urease is prepared by reacting acid urease with glutaraldehyde to form covalent bonds therebetween. The acid ureases used were ureases similar to those described in U.S. Patent Nos. 4,753,882, 4,970,153, and 5,093,255, each of which are incorporated herein by reference. Immunization with the inactive antigen was effected and infections caused by H. pylori were prevented or alleviated.

The antigen is preferably administered by oral or systemic methods.

According to another aspect of the invention, there is provided an oral vaccine comprising an antigen including one of a glutaraldehyde inactivated acid urease and a formaldehyde inactivated acid urease effective for the treatment of active Helicobacter pylori infections in humans or animals.

Many pathogenic organisms gain entrance to the body through the mouth. In such cases, mucosal immunization may offer extra protection to injection which could be even more effective than systemic immunization alone. The immunoglobulin A induced by gastrointestinal associated lymphoid tissue (GALT) and nasopharyngeal associated lymphoid tissue (NALT) may offer mucosal immunity that is not always induced by systemic immunization.

Oral immunization offers an advantage in that in some animals and man, systemic immunization does not always produce mucosal immunity. There are other advantages to oral immunization, some of which are of considerable economic importance. One such advantage is that oral immunization is less expensive than systematically injected antigens. This is especially important in mass immunization and immunizations performed in third world countries where sterile techniques and medically trained personnel are not always available.

As attractive as oral immunization may be, there are numerous problems inherent in oral usage. Only a very small fraction of the administered macromolecular antigens are absorbed which necessitates the administration of excessive doses of antigen if the oral route is chosen. If even a minuscule amount is to reach the lymphoid tissue in the sub mucosa or in Peyer's Patches, oral immunization may nonetheless be impractical if the antigen is a toxic bacteria. It would be hazardous to give large quantities of live toxic bacteria which might result in ulceration of the mucosa and even systemic toxicity. For this reason, it is often necessary to use an attenuated toxin or bacteria usually produced from less toxic recombinant derivatives of toxic organisms. For instance, immunity was induced in humans using a recombinant derivative of Salmonella typhi. (Infection and Immunity 60:2023-2029, 1992). In the aforementioned study, oral immunization was more effective in producing secretory IgA from the intestinal mucosa than it was in producing systemic immunity as measured by serum antibody production.

The absorption of macromolecules and particulate matter across the intact gastrointestinal tract has been studied extensively. (Digestive Disease 8(3):169-172, 1990). Macromolecules can cross the intestinal barrier at tight junctions between mucosal cells. (Gastoenterol:100:719-724, 1991). Particulate matter passes through the intact mucosa by endocytosis. The cells which perform this function (formally called lymph epithelial cells) are really specialized cells similar to reticuloendothelial cells elsewhere. Such cells are found in the lymphoid aggregates of Peyer's Patches in the distal ilium and in addition are widely distributed individually throughout the G.I. tract. Each cell is associated with a small collection of lymphocytes in the adjacent submucosa. These cells are called microfold cells are usually designated as M cells. M cells absorb particulate material and macromolecules similar to the phagocytosis of particulate material by the Kupffer cells in the liver. (Gastroenteric Cline N A 20(3):531547, 1991). The process by which this particulate matter passes into cells is called pinocytosis. Some pathogens actually enter the body through the GI tract by pinocytosis. For example, Monilia is known to cross the intact intestinal mucosa by pinocytosis.

Another problem presented by oral immunization is digestion of the antigen by the intestinal enzymes.

In accordance with a preferred embodiment of the invention therefore the antigen is encapsulated eg. in a digestant resistant material, so that the antigens can reach GALT inside of a particle. However, if the antigen is inside a bacteria it is already protected by the bacterial wall.

Many encapsulants have been found to be satisfactory in accordance with this aspect of the invention.

According to one preferred aspect of the invention there is provided an oral vacuum comprising an antigen including one of a glutaraldehyde inactivated acid urease and a formaldehyde inactivated acid urease effective for the treatment of active Helicobacter pylori infections in humans or animals; and
a peptic digestion resistant material for encapsulating said antigen.

Also according to the present invention there is provided an oral vacuum comprising;
an antigen including one of a glutaraldehyde inactivated acid urease and a formaldehyde inactivated acid urease effective for inducing mucosal immunity or systemic immunity to infection by H. pylori in humans and comparable Helicobacter infections in animals and livestock; and
a peptic digestion resistant material for encapsulating said antigen.

Further, according to the present invention there is provided an oral vaccine comprising:
a non-enzymatic urease effective for inducing at least one of systemic immunity and mucosal immunity to infection by H.pylori in animals, including humans; and
a peptic digestion resistant composition for encapsulating quantities of said non-enzymatic acid urease of a size sufficient to permit absorption thereof in the gastrointestinal tract to effect immunity.

Preferably, the non-enzymatic urease is selected from the group consisting of glutaraldehyde inactivated acid ureases, formaldehyde inactivated acid ureases, and inactivated acid ureases having a mean molecular weight ranging up to 2,000,000 Daltons, preferably in the range of 250,000 to 2,000,000 Daltons.

According to yet a further aspect of the invention there is provided an oral vacuum comprising:
a non-enzymatic acid urease effective for treating active infections of H.pylori in animals, including humans;
said non-enzymatic acid urease having a molecular weight sufficient to permit recognition by M cells for the production of antibodies; and
a peptic digestion resistant composition for encapsulating quantities of said non-enzymatic acid urease of a size sufficient to permit absorption thereof in the gastrointestinal tract to render the H. pylori non-pathenogenic.

Preferably at least one non-enzymatic acid urease is selected from the group consisting of glutaraldehyde inactivated acid ureases, formaldehyde inactivated acid ureases, and inactivated acid ureases having a mean molecular weight ranging up to 2,000,000 Daltons, preferably in the range of 250,000 to 2,000,000 Daltons.

Also according to this invention there is provided a composition of matter, which comprises, in combination:
a non-enzymatic acid urease antigen synthesized by bonding jack bean urease with glutaraldehyde, wherein said non-enzymatic acid urease antigen stimulates production of antiurease effective for rendering endogenous H.pylori non-pathenogenic; and
means for encapsulating said non-enzymatic urease antigen with a peptically resistant material.

Further, the present invention comprises use of an inactivated urease having a mean molecular weight ranging up to 2,000,000 Daltons, preferably in the range of 250,000 to 2,000,000 Daltons for the manufacture of an oral vaccine for administering to an animal in a physiologically innocuous dosage form for treating and/or preventing in animals including humans, infections by H.pylori and comparable Helicobacter infections,
said inactivated urease being microencapsulated in a peptic digestion resistant composition in quantities of said non-enzymatic acid urease of a size sufficient to permit absorption thereof in the gastrointestinal tract to render the H.pylori non-pathenogenic.

Preferably the inactivated urease is selected from the group of non-enyzmatic ureases consisting of glutaraldehyde inactivated urease and formaldehyde inactivated urease.

One useful inactivated urease is inactivated jack bean urease.

The invention includes orally treating bacterial ureases in humans, wherein
said urease enzyme is inactivated by inducing covalent bonding to increase a molecular weight of said acid urease; and is micro-encapsulated in digestion resistant material in quantities sufficient to permit absorption thereof in the gastrointestinal tract; and is administered in a physiologically innocuous dosage form, a therapeutically effective amount to stimulate production of antiurease antibodies.

To protect material to the cells and other RE cells lining the oral pharynx and GI tract, the antigen is preferably encapsulated in a biodegradable plastic, such as the polygalactides, polyactides, and polyglycolides. One successful material has been found to be poly (DL lactide-co-glycolide) DL PLG which is an acid impermeable polyester that has been used in absorbable surgical devices. Other materials have been found to be satisfactory and Dextrans and other hydrophilic colloids have been utilized and described. one type of very satisfactory encapsulating method is by liposomes. These are spherical blisters of lipid material having a single or multi-layers of lipid as a surrounding capsule. Because of their lipid soluble surfaces these liposomes are adherent to M cells and easily endocytosed by them. If the antigen is contained within a bacteria, further encapsulation is not required and the bacteria may be ingested to produce immunity.

A number of acid ureases including Helicobacter urease were inactivated as described in a U.S. Patent Application titled "A Method for Treating Gastritis and Peptic Ulcers by Inhibiting Helicobacter Urease", filed November 18, 1993, by Harry H. LeVeen et al, which is incorporated herein by reference. Furthermore, other acid ureases are described in U.S. Patent Nos. 4,753,882, 4,970,153 and 5,093,255, which are incorporated herein by reference. The preparations of the above referenced application were made specifically for the immunization of humans and animals to Helicobacter urease for either the prevention or treatment of Helicobacter infections. The non enymatic urease antigen was encapsulated in DL PLG as described in the literature. (Controlled Release 1990; 11:205-14; Mol. Immunol 1991; 28:287-94 herein incorporated by reference) This antigen was injected into laboratory mice in oral doses of 0.25 milligrams. Satisfactory titres to urease as demonstrated by ELISA and tanned red cell agglutination tests were obtained in laboratory rodents. Orally immunized mice, were protected from a challenge with H. felis as described in the literature. The infection in mice initially colonized by H. felis was rapidly eliminated from mice within a two week period.

Adjuvants are also used with vaccines to enhance the immunogenic potential of antigen either by stimulating the production of antibodies by lymphocytes or by aiding in the delivery of the antigen. Some substances such the B subunit of Cholera toxin have a special affinity for M cells and adhere to the surface of these cells. This aids in absorption and delivery of the antigen and may stimulate the production of immune globulin. Some of substances such as BCG and fractions thereof have been used as vaccine adjuvants. The subject of adjuvants has been recently covered in a book on this subject. (Immunopharmacology of Infectious Diseases Vaccine Adjuvants and Modulators of Non-Specific Resistance ALAN LISS, NEW YORK 1987).

While antibiotics fulfill an important role in treating acute diseases caused by bacterial infections they have little value in prophylaxis against infection and in the treatment of chronic illnesses. Long term antibiotic therapy is often complicated by resistant organisms, toxicity, and non-compliance. Therefore, the role of vaccines has again assumed extreme importance for preventing and treating chronic or recurrent diseases. One such chronic disease has been duodenal ulcer.

Duodenal ulcer is a common disease which has an extremely high recurrence rate which has more recently been shown to be caused by a bacterial infection with an organism known as *Helicobacter pylori*. These bacteria are potent producers of an extracellular acid urease. The extracellular acid urease breaks down urea to form a protective shield of ammonia around the *H. pylori* which also solubilizes the protective mucin coating of the stomach. Because urease is a toxic substance it has not been used for oral immunization.

To avoid the problems of encapsulation and expensive chemical inactivation of urease, the present invention provides a non-toxic bacteria containing a high concentration of intracellular acid urease has been chosen to immunize patients to urease via the oral route.

Briefly stated yet a further aspect of the present invention provides a method to immunize animals and humans to urease utilizing an oral vaccine and a method for the manufacture of the vaccine is provided by the present invention. The oral vaccine includes an intracellular urease antigen contained in dead or live bacteria. The intracellular position of the urease antigen protects it from the destructive action of digestive enzymes which are normally present in the gastrointestinal tract. The dead or live bacteria are transferred across the G.I. tract as particulate matter by M cells and reach R.E. cells and lymphoid elements which produce antibodies. These antibodies inactivate the enzymatic activity of urease and confer systemic and mucosal immunity to the enzymatic activity of urease. One embodiment of the invention utilizes dead *Lactobacillus fermentum* bacteria having enzymatically active intracellular urease which produces effective immunity to urease which in turn inhibits ammonia production from urea in the immunized animal. The intracellular urease may be optionally inactivated by treating the *Lactobacillus fermentum* with dilute formaldehyde or glutaraldehyde. The bacteria are killed by exposure to hot alcohol and are then dried. The dried material is administered to effect urease immunization. The treatment is effective in the prevention or elimination of infections of *Helicobacter* *pylori* in humans and animals. The bacteria is further effective for preventing urea turnover in cirrhotics to alleviate hepatic encephalopathy and preventing urea breakdown in livestock.

The present invention provides a method of oral immunization to urease and inhibition of urea turnover in humans and animals comprising orally administering one of suitable killed and live bacteria containing one of active and inactivated intracellular urease.

Furthermore, the present invention provides a method and an oral urease vaccine comprising dead Lactobacillus fermentum containing acid urease effective to produce immunity to urease for preventing infection with *Helicobacter pylori* in animals and livestock.

Still further, the present invention provides a method and an oral urease vaccine for one of preventing urea turnover in cirrhotics to alleviate hepatic encephalopathy and preventing urea breakdown in livestock wherein the oral vaccine includes killed Lactobacillus fermentum.

Still another feature provided by the present invention is a method and an oral urease vaccine effective for one of the treatment of active Helicobacter infections in humans and animals and the prevention of urea turnover in humans and animals wherein the oral vaccine includes dead Lactobacillus fermentum having an intracellular inactivated acid urease inactivated by one of dilute formaldehyde and glutaraldehyde.

Yet another feature of the present invention is a method of producing an oral urease vaccine including dead Lactobacillus fermentum having an intracellular inactivated acid urease effective for one of the treatment of active Helicobacter infections in humans and animals and the prevention of urea turnover in humans and animals comprising: condensing a culture of Lactobacillus fermenturn and removing an effluent thereof to produce a solid; pasteurizing the solid in a solvent in order to kill the Lactobacillus fermentum included in the solid; removing the solvent from the solid; and treating the Lactobacillus fermentum with one of dilute formaldehyde and glutaraldehyde in order to inactivate urease contained in the Lactobacillus fermentum.

*Helicobacter pylori* has been identified in 90% of the patients with duodenal ulcers not caused by medications such as non-steroidal anti-inflammatory agents. Most people believe that *H. pylori* is not the sole cause of ulcers since duodenal ulcer formation does occur in the absence of acid. (Gastroenterol. 96:615-25, 1989) For this reason, H2 receptor antagonists and proton pump inhibitors are still effective in the treatment of ulcers together with the eradication of the *H. pylori* organism. It has been amply demonstrated in experimental animals that urease produced by *H. pylori* is essential for colonization in experimental animals. Mutants of *H. pylori,* which do not produce urease but are immunologically identical to the original strain, do not produce the typical gastritis when injected into laboratory animals. Colonization in the gastric mucosa does not occur. (RID (Suppl 8) 13:S681-S685, 1991). Not only has *H. pylori* been implicated in peptic ulceration but also in gastric carcinoma. (Scand J Gastroenterol 26 suppl) 181:51-57, 1991)

Urease is a highly toxic enzyme and is lethal when injected into all known species at 50 Sumner units per kilogram. Large quantities of urease can produce tissue necrosis, ulceration, and even death. Therefore it is essential that the urease be rendered non-enzymatic. This has been accomplished by treating the bacteria with dilute glutaraldehyde as described in U.S. Patent No. 4,837,017, which is incorporated herein by reference. However, it has been found that inactivation of the intracellular urease is unnecessary except in situations where the native enzyme is injected to induce systemic immunity.

In one aspect of the present invention a urease enzyme is produced by growing Lactobacillus fermentum in batch culture. Lactobacillus fermentum is a non-toxic bacteria found extensively in food such as dairy products, sour dough, sausages and pickles. It has also been isolated from the human G.I. tract. The culture is filtered and condensed to concentrate the bacteria and effluent is discarded. The solid is then pasteurized using hot alcohol which kills the bacteria. The ethanol is then removed from the product by vacuum distillation and the product is dried. The dried product consists of dead bacteria containing 3.5 units of urease per milligram. (One unit is defined as the amount of enzyme that releases 1 micromole of ammonia from urea per minute at pH 4.0 and at 37 degrees C.). The dried product is then orally administered in order to effect urease immunization.

Such an antigen is also used to prevent ethyl carbamate formation by destroying urea formed during the fermentation of wine and is described in U.S. Patent No. 4,970,153 issued on Nov. 13, 1990. A suitable commercial product for use in the wine industry is available from Enzyme Development Corporation, 2 Penn Plaza, Suite 2439, New York, NY 10121-0034. A similar product is available from Takeda USA Inc., Orangeburg, NY 10962-2614.

The invention will now be described by reference to the following examples which are offered to further explain and exemplify the present invention.

### Example 1

Acid urease is obtained from culture of Lactobacillus fermentation TK 1214. This material is purified by affinity chromatography according to a method known in the art and described by Wong and Shobe in Single Step Purification of Urease by Affinity Chromatography, Can J. Microbial 20:623-630, 1974. The purified urease is then reacted with glutaraldehyde, as described in the above referenced U.S. Patent No. 4,837,017, to form a non-enzymatic urease antigen. Immunized animals showed a complete regression of gastric lesions as compared to normal control animals. Mice immunized with the inactive urease antigen prior to the challenging dose of Helicobacter felis are protected from the development of gastritis. The infecting organism could not be recovered from the gastric content and was not present on histological examination of gastric tissue.

### EXAMPLE 2

This above described dead Lactobacillus fermentum bacteria is fed to laboratory mice in oral doses of 0.1 mg. suspended in 0.4 ml of 1% sodium bicarbonate solution and injected into the stomach using a feeding needle. In tests conducted the dose is given at days 1 and 3. At day 7, the dose is increased to 0.25 mg. In the test, after two weeks, satisfactory titres to urease, as demonstrated by ELISA, and tanned red cell agglutination tests were obtained in laboratory rodents. Orally immunized mice, were protected from a challenge with H. felis as described in published literature. (Scand. J. Gastroenterol 26:909-915, 1991) The infection in mice initially colonized by H. felis was rapidly eliminated from the mice within a two week period.

Having described preferred embodiments of the invention it is to be understood that the invention is not limited to those precise embodiments, and that various changes and modifications may be effected therein by one skilled in the art without departing from the scope or spirit of the invention as defined in the appended claims.

## Claims

**1.** Use of a non-enzymatic urease antigen for the manufacture of a medicament for treating and/or preventing, in animals, including humans, infections, by Helicobacter pylori and comparable Helicobacter infections.

**2.** Use as claimed in claim 1, wherein the treatment comprises immunization.

**3.** Use as claimed in claim 1 or 2, wherein the non-enzymatic urease antigen is an acid urease of bacterial origin.

**4.** Use as claimed in claim 1 or 2, wherein the non-enzymatic urease antigen urease is a urease of plant origin.

**5.** Use as claimed in any one of the preceding claims, wherein said non-enzymatic urease antigen has a urease inactivated by covalent bonding to increase a molecular weight thereof.

**6.** Use as claimed in any one of the preceding claims, wherein said non-enzymatic urease antigen is a glutaraldehyde or formaldehyde inactivated acid urease.

**7.** Use as claimed in any one of the preceding claims, wherein jack bean urease is inactivated by covalent bonding to produce said non-enzymatic urease antigen wherein said non-enzymatic urease antigen has a mean molecular weight ranging up to 2,000,000 Daltons, preferably in the range of 250,000 to 2,000,000 Daltons.

**8.** Use as claimed in any one of claims 1 to 6, wherein urease produced by a microorganism of the genus Lactobacillus is inactivated by covalent bonding to produce said non-enzymatic urease antigen wherein said non-enzymatic urease antigen has a mean molecular weight ranging up to 2,000,000 Daltons, preferably in the range of 250,000 to 2,000,000 Daltons.

**9.** Use as claimed in claim 8, wherein said microorganism is one of Lactobacillus fermentum, Lactobacillus reuteri and Lactobacillus ruminis.

**10.** Use as claimed in claim 8, wherein said microorganism is Lactobacillus fermentum TK 1214.

**11.** Use as claimed in claim 8, wherein said microorganism is Bacillus TB-90.

**12.** Use as claimed in any one of claims 1 to 6, wherein urease produced by a microorganism of the genus Streptcooccus is inactivated by covalent bonding to produce said non-enzymatic urease antigen wherein said non-enzymatic urease antigen has a mean molecular weight ranging up to 2,000,000 Daltons preferably in the range of 250,000 to 2,000,000 Daltons.

**13.** Use as claimed in claim 12, wherein said microorganism is one of Streptococcus bovis, Streptococcus Reuteri and Stretpococcus salivarius.

**14.** Use as claimed in any one of the preceding claims, wherein said non-enzymatic urease antigen induces mucosal or systemic immunity to infection by H. pylori in humans and comparable Helicobacter infections in animals and livestocks.

**15.** Use as claimed in any one of the preceding claims, wherein said antigen is encapsulated.

**16.** Use as claimed in claim 15, wherein said antigen is encapsulated in a peptic digestion resistant material.

**17.** An oral vaccine comprising an antigen including one of a glutaraldehyde inactivated acid urease and a formaldehyde inactivated acid urease effective for the treatment of active Helicobacter pylori infections in humans or animals; and
a peptic digestion resistant material for encapsulating said antigen.

**18.** An oral vaccine comprising;
an antigen including one of a glutaraldehyde inactivated acid urease and a formaldehyde inactivated acid urease effective for inducing mucosal immunity or systemic immunity to infection by H. pylori in humans and comparable Helicobacter infections in animals and livestock; and
a peptic digestion resistant material for encapsulating said antigen.

**19.** An oral vaccine comprising:
a non-enzymatic urease effective for inducing at least one of systemic immunity and mucosal immunity to infection by H.pylori in animals, including humans; and
a peptic digestion resistant composition for encapsulating quantities of said non-enzymatic acid urease of a size sufficient to permit absorption thereof in the gastrointestinal tract to effect immunity.

**20.** An oral urease vaccine as claimed in claim 17 or 18, wherein said non-enzymatic urease is selected from the group consisting of glutaraldehyde inactivated acid ureases, formaldehyde inactivated acid ureases, and inactivated acid ureases having a mean molecular weight ranging up to 2,000,000 Daltons, preferably in the range of 250,000 to 2,000,000 Daltons.

**21.** An oral vaccine comprising:
a non-enzymatic acid urease effective for treating active infections of H.pylori in animals, including humans;
said non-enzymatic acid urease having a molecular weight sufficient to permit recognition by M cells for the production of antibodies; and
a peptic digestion resistant composition for encapsulating quantities of said non-enzymatic acid urease of a size sufficient to permit absorption thereof in the gastrointestinal tract to render the H. pylori non-pathenogenic.

**22.** An oral vaccine as claimed in claim 21, wherein said at least one non-enzymatic acid urease is selected from the group consisting of glutaraldehyde inactivated acid ureases, formaldehyde inactivated acid ureases, and inactivated acid ureases having a mean molecular weight ranging up to 2,000,000 Daltons, preferably in the range of 250,000 to 2,000,000 Daltons.

**23.** A composition of matter, which comprises, in combination:
a non-enzymatic acid urease antigen synthesized by bonding jack bean urease with glutaraldehyde, wherein said non-enzymatic acid urease antigen stimulates production of antiurease effective for rendering endogenous H.pylori non-pathenogenic; and
means for encapsulating said non-enzymatic urease antigen with a peptically resistant material.

**24.** Use of an inactivated urease having a mean molecular weight ranging up to 2,000,000 Daltons, preferably in the range of 250,000 to 2,000,000 Daltons for the manufacture of an oral vaccine for administering to an animal in a physiologically innocuous dosage form for treating and/or preventing in animals including humans, infections by H.pylori and comparable Helicobacter infections,
said inactivated urease being microencapsulated in a peptic digestion resistant composition in quantities of said non-enzymatic acid urease of a size sufficient to permit absorption thereof in the gastrointestinal tract to render the H.pylori non-pathenogenic.

**25.** Use as claimed in claim 24, wherein said inactivated urease is selected from the group of non-enyzmatic ureases consisting of glutaraldehyde inactivated urease and formaldehyde inactivated urease.

**26.** Use as claimed in claim 24 or 25, wherein said inactivated urease is inactivated jack bean urease.

**27.** Use as claimed in any one of claims 24 to 26, for orally treating bacterial ureases in humans, wherein
said urease enzyme is inactivated by inducing covalent bonding to increase a molecular weight of said acid urease; and is micro-encapsulated in digestion resistant material in quantities sufficient to permit absorption thereof in the gastrointestinal tract; and is administered in a physiologically innocuous dosage form, a therapeutically effective amount to stimulate production of antiurease antibodies.

**28.** Use as claimed in any one of the preceding claims, wherein a therapeutically effective amount of a suitable live or killed bacteria is orally administered containing one of active and inactivated intracellular urease.

**29.** Use as claimed in claim 28 wherein said bacteria is Lactobacillus fermentum.

**30.** Use as claimed in claim 29, wherein said bacteria is dead Lactobacillus fermentum.

**31.** An oral vaccine containing a glutaraldehyde or formaldehyde inactivated acid ureases and or other ureases which induces mucosal immunity to infection by H. pylori in humans and comparable Helicobacter infections in animals and livestock.

**32.** An oral vaccine containing a glutaraldehyde or formaldehyde inactivated acid ureases and or other ureases which induces systemic immunity to infection by H. pylori in humans and comparable Helicobacter infections in animals and livestock.

**33.** An oral vaccine containing a glutaraldehyde or formaldehyde inactivated acid ureases and or other ureases for the treatment of active Helicobacter infections in humans or animals.

**34.** Use of a material for the manufacture of a medicament for oral immunization to urease and inhibition of urea turnover in animals, said material comprising a therapeutically effective amount of a suitable bacteria which is one of killed and live wherein said bacteria contains one of active and inactivated intracellular urease.

**35.** Use of a material for the manufacture of a medicament for immunization by oral administration, said material comprising a therapeutically effective amount of a urease vaccine including bacteria containing acid urease effective to produce immunity to urease for preventing infection with Helicobacter pylori in animals.

**36.** Use as claimed in claim 34 or 35, wherein said bacteria is Lactobacillus.

**37.** Use as claimed in any one of claims 34 to 36, wherein said animals are humans.

**38.** A preparation for oral administration for immunisation to urease and inhibition of urea turnover in animals, (including humans) comprising a therapeutically effective amount of a suitable bacteria which is one of live or dead, wherein the bacteria contains one of active and inactivated intracellular urease. 39. Use of a non-enzymatic urease antigen for treating and/or preventing, in animals (including humans), infections by Helicobacter pylori and comparable Helicobacter infections.

**40.** Use of an inactivated urease having a mean molecular weight ranging up to 2,000,000 Daltons, preferably in the range 250,000 to 2,000,000 Daltons, for treating and/or preventing in animals (including humans) infections by Helicobacter pylori and comparable Helicobacter infections.

**41.** Use of a material for oral immunization to urease and inhibition of urea turnover in animals, said material comprising a therapeutically effective amount of a suitable bacteria which is one of killed and live wherein said bacteria contains one of active and inactivated intracellular urease.
